Europäisches Patentamt

(19) European Patent Office

Office européen des brevets

(11) Veröffentlichungsnummer: **0 092 096**
**B1**

(12) **EUROPÄISCHE PATENTSCHRIFT**

(45) Veröffentlichungstag der Patentschrift:
03.07.85

(51) Int. Cl.⁴: **C 07 C 125/065**

(21) Anmeldenummer: 83103307.1

(22) Anmeldetag: 05.04.83

(54) Verfahren zum Herstellen von N-Methoxy-N-methyl-urethanen.

(30) Priorität: 14.04.82 DE 3213684

(43) Veröffentlichungstag der Anmeldung:
26.10.83 Patentblatt 83/43

(45) Bekanntmachung des Hinweises auf die Patenterteilung:
03.07.85 Patentblatt 85/27

(84) Benannte Vertragsstaaten:
AT BE CH DE FR GB IT LI NL

(56) Entgegenhaltungen:
CHEMICAL ABSTRACTS, Band 77, Nr. 13, 25. Oktober 1972, Seite 424, Nr. 88118t, Columbus, Ohio, USA

(73) Patentinhaber: BASF Aktiengesellschaft,
Carl-Bosch-Strasse 38, D-6700 Ludwigshafen (DE)

(72) Erfinder: Reissenweber, Gernot, Dr., Drosselstrasse 15,
D-6737 Boehl-Iggelheim (DE)
Erfinder: Schirmer, Ulrich, Dr., Berghalde 79,
D-6900 Heidelberg (DE)

### Beschreibung

N-Methoxy-N-methyl-urethane sind wichtige Vor- bzw. Zwischenprodukte zur Herstellung von Schädlingsbekämpfungsmitteln (vgl. C.A. Vol. 77, S. 424, Nr. 88118t (1972).

Die vorliegende Erfingung betrifft ein Verfahren zum Herstellen von N-Methoxy-N-methyl-urethanen, wobei man zunächst (1) Hydroxylamin mit einem Chlor-ameisensäure-alkylester in das entsprechende N-Hydroxy-urethan überführt, dann (2) das N-Hydroxy-urethan mit einem Methylierungsmittel zum N-Methoxy-N-methyl-urethan umsetzt, und schliesslich (3) das N-Methoxy-N-methyl-urethan isoliert.

Verfahren dieser Art sind bekannt, wozu als repräsentativ verwiesen werden kann auf A.T. Fuller u.H. King, Soc. *1947,* 963, sowie R.T. Major u. E.E. Fleck, Am. Soc. *50,* 1479 (1928).

Das Herstellen von N-Methoxy-N-methyl-urethanen nach den bekannten Verfahrensweisen lässt — namentlich für das Herstellen in technischwirtschaftlichem Massstab — noch eine Reihe von Wünschen offen: So ist es z.B. erforderlich, die einzelnen Verfahrensschritte (1), (2) und (3) als diskrete Einzeloperationen in jeweils voneinander getrennten Vorrichtungen durchzuführen, wobei hinzukommt, dass die Ausbeuten in Schritt (1) sowie insbesondere Schritt (2) mässig sind und bei Schritt (3) — als Destillation ausgestaltet — die Gefahr explosiver Zersetzung besteht. Der Versuch, das von Fuller u. King angegebene Verfahren nachzuvollziehen, führte nach zwei vergeblichen Ansätzen zu einem Ergebnis mit einer Ausbeute von etwa 60%, bei 85 bis 90%iger Reinheit, erreichte also nicht die Angabe der früheren Bearbeiter von 94%.

Die Aufgabenstellung, die zur vorliegenden Erfindung geführt hat, war, ein Verfahren der eingangs definierten Art aufzuzeigen, das mit den vorerwähnten Nachteilen nicht belastet ist.

Es wurde gefunden, dass die gestellte Aufgabe gelöst werden kann, wenn man die Verfahrensschritte (1), (2) und (3) in einem durchgehenden Zug ausführt, mit den Massgaben, dass man in Schritt (2) mit einem Zweiphasen-System aus einer flüssigen wässrigen Phase sowie einer flüssigen nicht-wässrigen Phase unter Einsatz eines Phasentransferkatalysators arbeitet und in Schritt (3) das N-Methoxy-N-methyl-urethan extraktiv isoliert.

Gegenstand der vorliegenden Erfindung ist dementsprechend ein Verfahren zum Herstellen von N-Methoxy-N-methyl-urethanen, wobei man zunächst (1) Hydroxylamin mit einem Chlor-ameisensäure-alkylester in das entsprechende N-Hydroxy-urethan überführt, dann (2) das N-Hydroxy-urethan mit einem Methylierungsmittel zum N-Methoxy-N-methyl-urethan umsetzt, und schliesslich (3) das N-Methoxy-N-methyl-urethan isoliert. Das erfindungsgemässe Verfahren ist dadurch gekennzeichnet, dass man zunächst

(1) unter ständigem Aufrechterhalten einer Temperatur im Bereich von −10 bis 20, vorzugsweise 0 bis 10°C sowie eines pH-Wertes im Bereich von 2 bis 7, vorzugsweise 4,5 bis 6,5,

(1.1) eine 0,5 bis 3, vorzugsweise 1 bis 2 molare wässrige Lösung eines anorganischen Hydroxylammonium-salzes mit

(1.2) der äquimolaren Menge eines Chlor-ameisensäure-alkylesters unter heftiger Durchmischung zusammenbringt, dann

(2) unter ständigem Aufrechterhalten einer Temperatur im Bereich von 10 bis 50, vorzugsweise 20 bis 40°C, sowie eines pH-Wertes im Bereich von 7 bis 14, vorzugsweise 8,5 bis 13,5

(2.1) ein Gemenge aus

(2.1.1) 100 Volumenteilen des gemäss (1) erhaltenen Reaktionsgemisches,

(2.1.2) 10 bis 50, vorzugsweise 15 bis 30 Volumenteilen eines in Wasser nicht löslichen organischen Lösungsmittels sowie

(2.1.3) 0,1 bis 2, vorzugsweise 0,2 bis 0,5 Mol.-% — bezogen auf das im Reaktionsgemisch unter (2.1.1) erhaltene N-Hydroxy-urethan — eines Phasentransferkatalysators, mit

(2.2) 100 bis 150, vorzugsweise 100 bis 120 Äquivalent-% — bezogen auf das im Reaktionsgemisch unter (2.1.1) enthaltene N-Hydroxy-urethan — eines in Wasser nicht löslichen, in dem Lösungsmittel unter (2.1.2) löslichen Methylierungsmittels

unter heftiger Durchmischung zusammenbringt und das Zusammengebrachte 1 bis 5, vorzugsweise 2 bis 3 h auf einer Temperatur im Bereich von 10 bis 50, vorzugsweise 20 bis 40°C hält, und schliesslich

(3) das in dem gemäss (2) erhaltenen Reaktionsgemisch noch vorhandene Methylierungsmittel zerstört, aus dem so behandelten Gemisch die nicht-wässrige flüssige Phase (Extrakt) gewinnt und aus dieser durch Abtreiben des Lösungsmittels das N-Methoxy-N-methyl-urethan isoliert.

Das neue Verfahren erlaubt es, N-Methoxy-N-methyl-urethane — die bekanntlich wertvolle Zwischenprodukte, z.B. für die Herstellung von Herbiziden, sind — in hoher Ausbeute und ungefährlich in technisch guter Reinheit zu gewinnen.

Zum dem erfindungsgemässen Verfahren ist im einzelnen das folgende zu sagen:

Die Durchführung des Verfahrens ist für den Fachmann mit keinen Schwierigkeiten verbunden; auch nicht mit apparativen: Man kann z.B. ohne weiteres in gängigen Rührapparaturen arbeiten, die mit einer Vorrichtung zum Kühlen bzw. Heizen versehen sind und Vorrichtungen zur Zu- und Abfuhr von Flüssigkeiten bzw. Gasen aufweisen.

Bei der Durchführung von Verfahrensschritt (1) hat es sich als zweckmässig erwiesen, die wässrige Lösung (1.1) des anorganischen Hydroxylammonium-salzes, das insbesondere ein Sulfat sein kann, vorzulegen und dann den gewünschten pH-Wert einzustellen; — wobei letzteres am wirtschaftlichsten durch Zugabe einer wässrigen Alkalilauge, wie insbesondere Natronlauge, erfolgen kann. Günstig ist es dann im weiteren, unter kräftigem Rühren den Chlor-ameisensäure-alkylester (1.2), der vorzugsweise ein $C_1$- bis $C_4$-Alkylester, und insbesondere der Methyl- oder Ethylester sein kann, allmählich zuzugeben, wobei die Temperatur durch Kühlung auf den erforderlichen Werten gehalten wird, ebenso wie der pH-Wert; – was durch fortlaufende Zugabe einer wässrigen Base, vorzugsweise in Form einer Alkalilauge, wie insbesondere Natronlauge, erfolgen kann.

Den Verfahrensschritt (2) kann man zweckmässigerweise einleiten, indem man zunächst das Gemenge (2.1) aus dem gemäss (1) erhaltenen Reaktionsgemisch (2.1.1), dem in Wasser nicht löslichen organischen Lösungsmittel (2.1.2) sowie dem Phasentransferkatalysator (2.1.3) herstellt. Hierbei ist zum Lösungsmittel (2.1.2) sowie Katalysator (2.1.3) zu bemerken, dass sie die einschlägig üblichen sein können. Als geeignete Lösungsmittel (2.1.2) sind z.B. zu nennen insbesondere chlorierte Kohlenwasserstoffe, wie Methylenchlorid, Chloroform, Tetrachlorkohlenstoff, 1,2-Dichlorethan und Gemische davon, sowie ferner aliphatisch-aromatische Kohlenwasserstoffe, wie Toluol, Ethylbenzol, o-, m-, p-Xylol, Isopropylbenzol und Gemische aus diesen. Als Phasentransferkatalysatoren (2.1.3) kommen vor allem in Betracht quartäre Ammoniumsalze wie N,N-Dibenzyl-N,N-dimethylammoniumchlorid, N-Benzyl-N,N,N-trimethylammoniumchlorid, N-Benzyl-N,N,N-triethylammoniumchlorid, N-Dodecyl-N,N,N-trimethylammoniumchlorid sowie ferner quartäre Phosphoniumsalze wie Tetraphenylphosphoniumbromid.

Nach dem Herstellen des Gemenges (2.1) wird man im allgemeinen dieses auf die für die weitere Umsetzung gewählte Temperatur sowie den gewählten pH-Wert bringen; – wobei letzteres in Analogie zu Schritt (1) erfolgen kann. Mit Erfolg lässt sich dann so weiter verfahren, dass man unter kräftigem Rühren das Methylierungsmittel (2.2) zugibt und im weiteren die Temperatur sowie den pH-Wert auf den erforderlichen Werten hält; – wobei letzteres wiederum durch fortlaufende Zugabe einer wässrigen Base erfolgen kann. Zur stofflichen Seite ist hier zu sagen, dass das Methylierungsmittel ein einschlägig übliches sein kann, also vornehmlich Dimethylsulfat oder auch ein Methylhalogenid wie Methyl-chlorid, -bromid oder -jodid. Ein nämliches gilt für die Base: Hier ist aus wirtschaftlichen Gründen an erster Stelle zu nennen Natronlauge, sowie ferner Natriumcarbonat. Nachdem man das Gemenge (2.1) und das Methylierungsmittel (2.2) zusammengebracht hat, wird das Zusammengebrachte dann noch – sinnvollerweise unter weiterem kräftigen Rühren – über die erforderliche Zeitspanne im erforderlichen Temperaturbereich gehalten.

Schritt (3) gilt der Aufarbeitung des in Schritt (2) gebildeten N-Methoxy-N-methyl-urethans. Hierzu zerstört man zunächst das im Reaktionsgemisch noch vorhandene Methylierungsmittel durch Umsetzung mit einem hierfür üblichen Mittel; – wobei man letzteres zweckmässigerweise so wählen sollte, dass ein wasserlösliches Umsetzungsprodukt resultiert, wie insbesondere bei der Verwendung von Ammoniak(-wasser). Danach trennt man die nicht-wässrige flüssige Phase vom Reaktionsgemisch ab, wächst sie gewünschtenfalls mit Wasser, treibt das Lösungsmittel ab und erhält als Rückstand das gewünschte N-Methoxy-N-methyl-urethan.

*Beispiel*

Es wurde gearbeitet mit einer gängigen Rührapparatur, die ein Nutzvolumen von 120 l hatte und mit einer Vorrichtung zum Kühlen bzw. Heizen versehen war sowie Vorrichtungen zur Zu- und Abfuhr von Flüssigkeiten bzw. Gasen aufwies.

(1) Erster Verfahrensschritt

In der Apparatur wurden 40 l einer 0,875 mol wässrigen Lösung von Hydroxylammoniumsulfat vorgelegt und durch Zugabe von 1,2 l einer 19 mol wässrigen Natronlauge auf einen pH-Wert von 6,5 gebracht. Hierauf wurde unter kräftigem Rühren im Verlauf von 150 min 7,8 kg, d.h. der stöchiometrischen Menge 98%iger Chlorameisensäureethylester zugegeben unter – durch Kühlung – ständigem Aufrechterhalten einer Temperatur im Bereich von 0 bis 10°C. Zugleich wurde der pH-Wert ständig im Bereich von 6,0 bis 6,8 gehalten durch fortlaufende Zugabe einer 19 mol wässrige Natronlauge (insgesamt 7,5 l).

(2) Zweiter Verfahrensschritt

Zu dem gemäss (1) erhaltenen Reaktionsgemisch, das ein Volumen von ca. 60 l (=100 Volumenteilen) hatte, wurden zugegeben 20 l (=33 Volumenteile) Methylenchlorid sowie 50 g (=0,3 Mol.-%, bezogen auf das im Reaktionsgemisch enthaltene N-Hydroxyurethan) Benzyltriethylammoniumchlorid als Phasentransferkatalysator. Hierauf wurde unter kräftigem Rühren eine Temperatur von 20°C eingestellt. Ebenfalls unter kräftigem Rühren wurde alsdann im Verlauf von 120 min 18,9 kg (=110 Äquivalent.-%, bezogen auf das im Reaktionsgemisch enthaltene N-Hydroxy-urethan) Dimethylsulfat zugegeben unter – durch Kühlung – ständigem Aufrechterhalten einer Temperatur im Bereich von 20 bis 35°C. Zugleich wurde der pH-Wert ständig im Bereich von 8,5 bis 13,5 gehalten durch fortlaufende Zugabe einer 19 mol wässrigen Natronlauge (insgesamt 7,5 l). Anschliessend wurde das Ganze unter weiterem kräftigen Rühren noch 3 h auf einer Temperatur von 30°C gehalten.

(3) Dritter Verfahrensschritt

Zu dem gemäss (2) erhaltenen Reaktionsgemisch wurden unter kräftigem Rühren 5 l 3 mol Ammoniakwasser zugegeben zur Zerstörung des noch vorhandenen Methylierungsmittels; anschliessend liess man das Reaktionsgemisch zur Ruhe kommen. Die sich hierbei unten absetzende nicht-wässrige flüssige Phase wurde abgezogen, mit 20 l Wasser gewaschen und durch Abtreiben (Maximaltemperatur 50°C) vom Lösungsmittel befreit. Es resultierte ein N-Methoxy-N-methyl-urethan (N-Methoxy-N-methyl-carbaminsäureethylester) in einer Ausbeute von 80% (=7,45 kg), bezogen auf Chlorameisensäureethylester sowie einer Reinheit von 96%.

## Patentanspruch

Verfahren zum Herstellen von N-Methoxy-N-methyl-urethanen, wobei man zunächst (1) Hydroxylamin mit einem Chlor-ameisensäure-alkylester in das entsprechende N-Hydroxy-urethan überführt, dann (2) das N-Hydroxy-urethan mit einem Methylierungsmittel zum N-Methoxy-N-methyl-urethan umsetzt, und schliesslich (3) das N-Methoxy-N-methyl-urethan isoliert, dadurch gekennzeichnet, dass man zunächst

(1) unter ständigem Aufrechterhalten einer Temperatur im Bereich von −10 bis 20°C sowie eines pH-Wertes im Bereich von 2 bis 7,

(1.1) eine 0,5 bis 3 molare wässerige Lösung eines anorganischen Hydroxylammonium-salzes mit

(1.2) der äquimolaren Menge eines Chlor-ameisensäure-alkylesters

unter heftiger Durchmischung zusammenbringt, dann

(2) unter ständigem Aufrechterhalten einer Temperatur im Bereich von 10 bis 50°C sowie eines pH-Wertes im Bereich von 7 bis 14,

(2.1) ein Gemenge aus

(2.1.1) 100 Volumenteilen des gemäss (1) erhaltenen Reaktionsgemisches,

(2.1.2) 10 bis 50 Volumenteilen eines in Wasser nicht löslichen organischen Lösungsmittels sowie

(2.1.3) 0,1 bis 2 Mol.-% — bezogen auf das im Reaktionsgemisch unter (2.1.1) erhaltene N-Hydroxy-urethan — eines Phasentransferkatalysators, mit

(2.2) 100 bis 150 Äquivalent-% — bezogen auf das im Reaktionsgemisch unter (2.1.1) enthaltene N-Hydroxy-urethan — eines in Wasser nicht löslichen, in dem Lösungsmittel unter (2.1.2) löslichen Methylierungsmittels

unter heftiger Durchmischung zusammenbringt und das Zusammengebrachte 1 bis 5 h auf einer Temperatur im Bereich von 10 bis 50°C hält, und schliesslich

(3) das in dem gemäss (2) erhaltenen Reaktionsgemisch noch vorhandene Methylierungsmittel zerstört, aus dem so behandelten Gemisch die nicht-wässerige flüssige Phase gewinnt und aus dieser durch Abtreiben des Lösungsmittels das N-Methoxy-N-methyl-urethan isoliert.

## Claim

A process for the preparation of an N-methoxy-N-methylurethane, in which first (1) hydroxylamine is converted with an alkyl chloroformate into the corresponding N-hydroxyurethane, then (2) the N-hydroxyurethane is reacted with a methylating agent to give the N-methoxy-N-methylurethane, and finally (3) the N-methoxy-N-methylurethane is isolated, wherein first,

(1) while constantly maintaining a temperature of from −10 to 20°C and a pH of from 2 to 7,

(1.1) a 0.5 to 3 molar aqueous solution of an inorganic hydroxylammonium salt is brought together, with vigorous mixing, with

(1.2) an equimolar amount of an alkyl chloroformate, then,

(2) while constantly maintaining a temperature of from 10 to 50°C and a pH of from 7 to 14,

(2.1) a mixture comprising

(2.1.1) 100 parts by volume of the reaction mixture obtained as described in (1),

(2.1.2) from 10 to 50 parts by volume of a water-insoluble organic solvent, and

(2.1.3) from 0.1 to 2 mole-%, based on the N-hydroxyurethane present in the reaction mixture under (2.1.1), of a phase-transfer catalyst, is brought together, with vigorous mixing, with

(2.2) from 100 to 150 equivalent-%, based on the N-hydroxyurethane present in the reaction mixture under (2.1.1), of a water-insoluble methylating agent which is soluble in the solvent under (2.1.2), and the resulting mixture is kept at from 10 to 50°C for from 1 to 5 hours, and finally,

(3) any methylating agent still present in the reaction mixture obtained as described in (2) is destroyed, the non-aqueous liquid phase is separated off from the mixture thus treated, and the N-methoxy-N-methylurethane is isolated from this phase by stripping off the solvent.

## Revendication

Procédé de préparation de N-méthoxy-N-méthyluréthannes, dans lequel, d'abord (1) on transforme de l'hydroxylamine, avec un ester alkylique d'acide chloroformique, en le N-hydroxy-uréthanne correspondant, puis (2) on fait réagir le N-hydroxy-uréthanne avec un agent de méthylation pour obtenir du N-méthoxy-N-méthyluréthanne et, enfin (3) on isole le N-méthoxy-N-méthyluréthanne, caractérisé par le fait que, d'abord,

(1) en maintenant constamment une température comprise entre −10 et 20°C et un pH de 2 à 7,

(1.1) on mélange, sous brassage intense, une solution aqueuse, de concentration molaire 0,5 à 3, d'un sel d'hydroxylammonium inorganique avec

(1.2) la quantité équimolaire d'un ester alkylique d'acide chloroformique, puis

(2) en maintenant constamment une température comprise entre 10 et 50°C et un pH compris entre 7 et 14,

(2.1) on réunit, sous brassage intense, un mélange de

(2.1.1) 100 parties en volume du mélange de réaction obtenu selon (1)

(2.1.2) 100 à 50 parties en volume d'un solvant organique non soluble dans l'eau ainsi que

(2.1.3) 0,1 à 2 mol-% — rapporté au N-hydroxy-uréthanne obtenu dans le mélange de réaction sous (2.1.1) — d'un catalyseur de transfert de phases, avec

(2.2) 100 à 150 équivalents-% — rapporté au N-hydroxy-uréthane obtenu dans le mélange de réaction sous (2.1.1) — d'un agent de méthylation, insoluble dans l'eau, soluble dans le solvant sous (2.1.2), et on maintient les parties réunies ci-dessus, pendant 1 à 5 h, à une température comprise entre 10 et 50°C et enfin

(3) on détruit l'agent de méthylation encore présent dans le mélange de réaction obtenu selon (2), on récupère du mélange ainsi traité la phase liquide non aqueuse et on isole de celle-ci, par entraînement du solvant, le N-méthoxy-N-méthyluréthanne.